# EUROPÄISCHE PATENTSCHRIFT

(11) **EP 0 896 667 B1**
(45) Veröffentlichungstag und Bekanntmachung des Hinweises auf die Patenterteilung: **11.07.2007**
(21) Anmeldenummer: 97921829.4
(22) Anmeldetag: 29.04.1997
(51) Int. Cl.: G01N 27/12

(54) **VERFAHREN ZUR DETEKTION OXIDIERBARER UND REDUZIERBARER GASE IN DER LUFT SOWIE VORRICHTUNG HIERZU**
PROCESS AND DEVICE FOR DETECTING OXIDABLE AND REDUCIBLE GASSES IN AIR
PROCEDE ET DISPOSITIF DE DETECTION DE GAZ OXYDABLES ET REDUCTIBLES DANS L'AIR

(30) Priorität: 30.04.1996 DE 19617297
(43) Veröffentlichungstag der Anmeldung: 17.02.1999
(73) Patentinhaber: Brand-Gerhart, Rosemarie, 63840 Hausen (DE)
(72) Erfinder: RUMP, Hanns, D-63840 Hausen (DE)
(74) Vertreter: Mierswa, Klaus
(86) Internationale Anmeldenummer: PCT/EP1997/002208
(87) Internationale Veröffentlichungsnummer: WO 1997/041423

(56) Entgegenhaltungen:
- US-A- 4 770 761
- US-A- 4 906 440
- US-A- 5 387 462
- GUTIERREZ F.J. ET AL: 'Design of polycrystalline gas sensors based on admittance spectrum measurement' SENSORS AND ACTUATORS B Bd. B07, Nr. 1-3, 30 September 1991, ELSEVIER SEQUOIA S.A., LAUSANNE, CH, Seiten 609 - 613, XP001098540
- WEIMAR U.; GÖPEL W.: 'A.c. measurements on tin oxide sensors to improve selectivities and sensitivities' SENSORS AND ACTUATORS B Bd. 26-27, 1995, Seiten 13 - 18
- KRAUSS A.; WEIMAR U.; GÖPEL W.: 'Impedanzspektroskopie an Halbleitergassensoren' TECHNISCHES MESSEN Bd. 62, Nr. 6, Juni 1995, Seiten 260 - 266, XP000527104
- SBERVEGLIERI G.: 'Recent developments in semiconducting thin-film gas sensors' SENSORS AND ACTUATORS Bd. 23, 1995, Seiten 103 - 109

## Beschreibung

### Technisches Gebiet:

Die Erfindung betrifft ein Verfahren zur Detektion oxidierbarer und reduzierbarer Gase in der Luft, nämlich NO₂ sowie CO, zum Zwecke der Steuerung von Lüftungseinrichtungen in Gebäuden oder Fahrzeugen und zum Zwecke der Überwachung von Verbrennungsprozessen oder von Abgaskatalysatoranlagen, unter Verwendung mindestens eines mit Kontaktelektroden versehenen, stromdurchflossenen und beheizbaren Sensors mit einem Sensormaterial aus Metalloxid sowie unter Verwendung einer elektrischen Auswerteschaltung, wobei der Sensor von Wechselstrom durchflossen wird, der zwischen mindestens zwei Frequenzen umgeschaltet wird, und einerseits die Änderung der Kapazitäten zwischen dem Sensormaterial und den Kontaktelektroden von der Auswerteschaltung als Merkmal der Anwesenheit von reduzierbaren Gasen und andererseits die Änderung der Kapazitäten innerhalb der Masse des Sensormaterials als Merkmal der Anwesenheit von oxidierbaren Gasen ausgewertet wird, wobei die unterschiedlichen Frequenzen mittels frequenzbestimmenden Bauteilen und mit einer Schwingschaltung erzeugt werden, der Sensor Bestandteil der Schwingschaltung ist, und die Schwingung der Schwingschaltung durch abwechselndes Umschalten der frequenzbestimmenden Bauteile zwischen mindestens den beiden Frequenzen abwechselnd verändert wird und die diesen Frequenzen zugeordneten Ausgangssignale der Schwingschaltung, wenn der Sensor von Normalluft umspült wird, zur Kalibration je einer Nulllinie herangezogen werden, wobei der höheren Frequenz der beiden Frequenzen stets reduzierbare Gase und der niedrigeren Frequenz der beiden Frequenzen stets oxidierbare Gase zugeordnet werden, und, wenn der Sensor von einem Gas abweichend von Normalluft umspült wird, sich die Ausgangssignale der Schwingschaltung gegenüber der jeweiligen Nulllinie verändern, wobei bei einer Abweichung der höheren Frequenz das Vorhandensein von reduzierbaren Gasen und bei einer Abweichung der niederen Frequenz das Vorhandensein von oxidierbaren Gasen im Bereich des Sensors nachgewiesen wird, und die jeweiligen Frequenzabweichungen als Maß für die Konzentration anwesender Gase herangezogen werden, gemäß dem Oberbegriff des Anspruchs 1. Die Erfindung betrifft ebenso eine Vorrichtung zur Durchführung des Verfahrens.

### Stand der Technik:

In zahlreichen Fällen ist es notwendig, Kenntnisse über Art und Konzentration von in der Atemluft anwesenden Gasen zu haben, um technische Steuer- und Regelprozesse ausführen zu können oder um Maßnahmen zum Schutz der Menschen vor gefährlichen oder unangenehmen, riechbaren Gasen einleiten zu können. Insbesondere zum Zwecke der bedarfs- und situationsgerechten Lüftung ist die Kenntnis des Gehalt an Schadstoffen innerhalb oder außerhalb des Aufenthaltsraumes von Menschen wichtig, um die Lüftung entweder einzuleiten oder aber bei außerhalb des zu belüftenden Bereiches vorhandenen Schadgas-Konzentrationen die Zufuhr von Außenluft zu unterbrechen.

Bei der Überwachung oder Regelung von Verbrennungsprozessen ist es wichtig, die Konzentration von unverbrannten Kohlenwasserstoffen oder von Kohlenmonoxid bzw. die Konzentration von Stickoxiden im Abgas zu kennen. Bei der Überwachung der Funktion von Katalysatoren in z. B. Kraftfahrzeugen ist es notwendig, die Konzentration von Gasen vor und nach dem Katalysator zu kennen, um den Wirkungsgrad des Katalysators bestimmen zu können.

Allen Beispielen ist es eigen, dass sowohl oxidierbare Gase, Kohlenwasserstoffe, Kohlenmonoxid, etc., als auch reduzierbare Gase, insbesondere Stickoxide, für die Beurteilung der jeweiligen Lage und zur Steuerung von Prozessen bzw. zum Einleiten von Maßnahmen zu detektieren sind.

Aufgrund der massenhaften Notwendigkeit solcher Sensoren sind Technologien nachgefragt, die keine großen Kosten verursachen. Sensoren auf der Basis polykristalliner Metalloxide sind kostengünstig und robust und in der Praxis erprobt. Allerdings bestehen derzeit zwar Erfahrungen in der Detektion oxidierbarer Substanzen, aber kaum Erfahrungen oder praktische Lösungen bei der Detektion sowohl oxidierbarer wie reduzierbarer Gase, die gleichzeitig auftreten und die gleichzeitig, also simultan, zu detektieren sind, ohne dass die Kosten und der apparative Aufwand im Sinne der Applikation zu teuer werden.

Metalloxidsensoren verändern grundsätzlich ihren elektrischen Widerstand bei Gasangebot. Die allgemein bekannten Sensoren bestehen aus einer beheizten und kontaktieren Schicht aus z. B. Zinndioxid oder einem anderen Metalloxid wie z. B. Zinkoxid, Galliumoxid, Wolframtrioxid, Aluminiumvanadat und anderen sensitiven Substanzen, wobei das Sensormaterial auf ein Substrat aus Keramik oder Silizium in Dünn- oder Dickfilmtechnik aufgebracht ist und Kontaktelektroden aufweist. Wenn eine oxidierbare gasförmige Substanz auf den Sensor trifft, gibt das Metalloxid Sauerstoff ab und wird insofern reduziert, wodurch sich der Leitwert erhöht. Weil sich das beheizte Metalloxid später mit dem Luftsauerstoff wieder verbindet, ist dieser Prozess reversibel. Da es sich um einen stetigen Austauschprozess zwischen angebotenem Gas, Metalloxid und Luftsauerstoff handelt und die Konzentration des Luftsauerstoffes quasi konstant ist, ist der Leitwert des Sensormaterials eine Funktion der Konzentration der anwesenden oxidierbaren Gase.

Bei reduzierbaren Gasen, wie Ozon, Stickoxiden etc., ist die elektrische Wirkung entgegengesetzt, weil es sich beim sensitiven Material des Sensors niemals um reines Metalloxid handelt, sondern dieses Metalloxid stets etwas anreduziert ist [SnO (2-x)]. Bei einer Begasung des Sensors wird somit der Leitwert insofern vermindert. Bei der gleichzeitigen Anwesenheit oxidierbarer und reduzierbarer Gase kommt es zu komplexen chemischen Reaktionen der Gase mit dem Sensormaterial sowie der Gase untereinander in der Nähe der heißen und mit katalytischen Materialien, wie z. B. Platin oder Palladium, versehenen Oberfläche des Sensors. In diesem Falle kann bisher eine eindeutige Aussage über die Konzentration der anwesenden Gase nicht gemacht werden.

Man hat beobachtet, dass die Reaktivität von Metalloxidsensoren gegenüber oxidierbaren oder reduzierbaren Gasen von der Temperatur abhängig ist. Bei geringen Temperaturen von z. B. <150 Grad C sind Zinndioxidsensoren nur gering empfindlich auf oxidierbare Gase und reagieren kaum mit einer Leitwerterhöhung bei Begasung. Treten aber reduzierbare Gase auf, reagiert der Sensor auf Begasung sofort mit einer signifikanten Leitwertverringerung. Aus diesem Grund ist vorgeschlagen worden, aus den verschiedenen Reaktionen des Sensors bei hohen (>300GradC) und niedrigen (<150GradC) Temperaturen entweder Sensorgruppen jeweils konstanter Temperatur zu schalten, die jeweils für eine Gasgruppe empfindlicher sind.

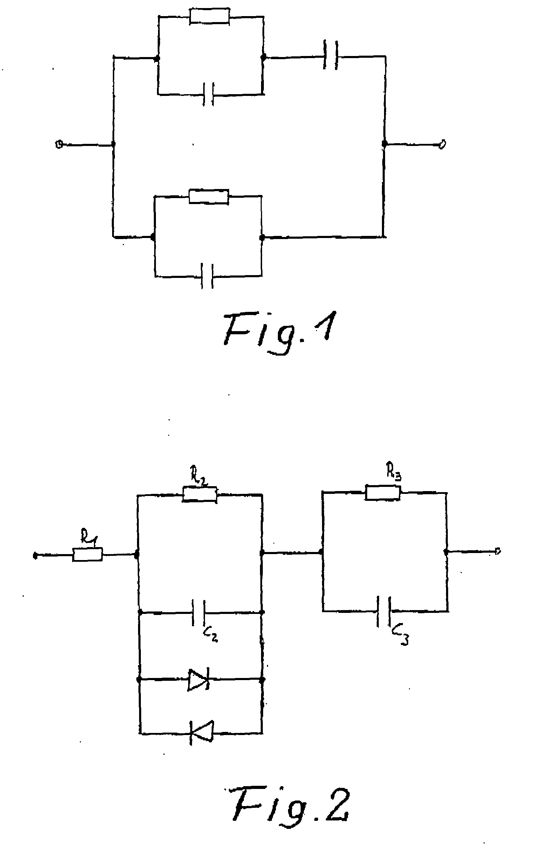

Aus der DE-A-38 27 426 ist es bekannt, die Temperatur des Sensors zu variieren und insofern mit einem einzigen Sensor die gewünschten Informationen zu erhalten. In der Praxis hat sich diese Methode nicht bewährt, weil bei niedrigen Temperaturen nach Begasung die Sensoren eine sehr lange Zeit benötigen, um sich auf einen Normalwert zu rekombinieren. Es ist weiter beobachtet worden, dass Metalloxidsensoren keineswegs aus einem gasabhängig veränderlichen ohmschen Widerstand bestehen, wie oft vereinfacht geschildert wird. Durch die DE-A-3917853 ist ein Verfahren bekannt, bei dem der Impendanzverlauf eines Sensorelements über die Frequenz eines Wechselstromes festgestellt und zur Identifizierung des angebotenen Gases genutzt wird. In der Literaturstelle "Sensors & actuators Bd. 4, 1991, Seiten 359-363" beschreibt J. Gutiérrez das elektrische Ersatzschaltbild eines Zinndioxidsensors (Figur 1) als eine Kombination von Widerständen und Kapazitäten und stellt fest, dass bei einem Gasangebot sich sämtliche Parameter ändern.

Aus der US-A-5 387 462 ist des Weiteren ein elektrisch reaktiver Verbundgegenstand bekannt geworden, welcher zufällige oder reguläre Felder von Mikrostrukturen aufweist, die partiell innerhalb einer einhüllenden Schicht eingeschlossen sind, wobei jede Mikrostruktur eine haarkristallähnliche Struktur und gegebenenfalls eine Deckschicht aufweist, welche die haarkristallähnliche Struktur umhüllt. Der Verbundgegenstand ist elektrisch leitend und dient als Komponente eines elektrischen Schaltkreises, einer Antenne, einer Mikroelektrode, als reaktives Heizelement oder als Multimode-Sensor, um die Gegenwart von Dämpfen, Gasen oder Flüssigkeiten nachzuweisen. Zur Messung wird bei der Anwesenheit des zu detektierenden Stoffes die Änderung der Orientierung der Haarkristalle ausgenutzt.

Durch die Literaturstelle Udo Weimar et al.: "A.c. measurements on tin oxide sensors to improve selectivities and sensitivities", Sensors & Actuators B, Vol.26-27, 1995, Seiten 13-18 ist ein dem Verfahren der eingangs genannten Gattung ähnliches Verfahren bekannt geworden. Hiernach ist es bekannt, dass das Empfindlichkeitsmaximum für ein oxidierbares Gas, wie H₂, auch bei Frequenzen liegen kann, die höher sind als diejenigen Frequenzen, bei denen das Empfindlichkeitsmaximum für ein reduzierbares Gas, wie NO₂, liegt. Die relative Lage der Empfindlichkeitsmaxima für oxidierbare und reduzierbare Gase hängt von vielen Faktoren ab, zum Beispiel von der speziellen Sorte des reduzierbaren bzw. oxidierbaren Gases (z.B. H₂ oder CO), von der Kontaktgeometrie, vom Sensormaterial, von der Schichtdicke, von der Schichtpräparation, der Korngröße, der Messtemperatur etc. Die bekannte Vorrichtung weist einen stromdurchflossenen und beheizten Sensor mit einem Sensormaterial aus Metalloxid sowie eine elektrische Auswerteschaltung auf, wobei der Sensor von Wechselstrom durchflossen wird, welcher entweder aus mindestens zwei Wechselströmen unterschiedlicher Frequenz besteht oder zwischen mindestens zwei Frequenzen umgeschaltet wird zur Messung der Konduktanz, nämlich von Realteil und Imaginärteil der Impedanz und einerseits die Änderung der Kapazitäten zwischen dem Sensormaterial und den Kontaktelektroden als Merkmal der Anwesenheit von reduzierbaren Gasen, wie NO₂, und andererseits und andererseits die Änderung der Kapazitäten innerhalb der Masse des Sensormaterials als Merkmal der Anwesenheit von oxidierbaren Gasen ausgewertet wird. Es werden bei einzelnen Frequenzen die von den sensorinternen Kapazitäten verursachten Phasenverschiebungen ermittelt und das Ausgangssignal in einen Imaginärteil und einen Realteil zerlegt, die in Bezug von bei Normalluft ermittelten Werten ein Maß für die Anwesenheit und Art von Gasen darstellen, wobei ebenfalls bei der Ermittlung von Phasenverschiebungen und daraus des Imaginär- und Realteils des Ausgangssignals hohe Frequenzen stets reduzierbaren Gasen und niedrige Frequenzen stets oxidierbaren Gasen zuordnet werden.

### Technische Aufgabe:

Aufgabe der Erfindung ist es, ein Verfahren und eine Vorrichtung zu schaffen, welches bzw. welche, aufbauend auf Sensoren mit einem Sensormaterial aus Metalloxid, beide Gasgruppen, nämlich oxidierbare wie auch reduzierbare Gase, mit ein und demselben Sensor gleichzeitig zu detektieren imstande sein sollen.

Ziel der Erfindung ist es, eine getrennte Aussage zur Veränderung des elektrischen Bahnwiderstandes und zur Veränderung des elektrischen Kontaktwiderstandes so zu treffen, dass eine Aussage zur Anwesenheit und zur Konzentration oxidierbarer und/oder reduzierbarer Gase mittels eines einzigen Sensors gemacht werden kann.

### Offenbarung der Erfindung und deren Vorteile:

Die Lösung der Aufgabe besteht verfahrensmäßig darin, dass der beheizte Sensor mit seinen Kontaktelektroden parallel der Schwingschaltung gelegt ist, deren Ausgangssignal auf den Mikroprozessor gegeben wird, wobei als frequenzbestimmende Bauteile zwei in Reihe geschaltete Kondensatoren dienen, von denen der zweite Kondensator auf den Eingang der Schwingschaltung gelegt ist und auf diesen arbeitet, und der erste Kondensator zur Veränderung der Eingangskapazität der Schwingschaltung abwechselnd oder periodisch kurzgeschlossen wird.

Eine Vorrichtung zur Durchführung des erfindungsgemäßen Verfahrens ist in Anspruch 4 definiert.

Der Vorteil des Verfahrens und der Vorrichtung hierzu bestehen darin, dass mittels ein und demselben Sensor, wie den bekannten Metalloxid-Sensoren, beide Gasgruppen, nämlich oxidierbare wie auch reduzierbare Gase, gleichzeitig detektiert werden können, wobei die Vorrichtung kostengünstig herstellbar ist.

Die Erfindung beruht auf der Beobachtung, dass die Reaktionsmechanismen bei oxidierbaren oder reduzierbaren Gasen, die elektrisch ausgewertet werden können, sich erheblich unterscheiden. Bei einem Angebot oxidierbarer Gase, zum Beispiel von CO, ändert sich der ohm'sche Leitwert des Masse des Sensormaterials als solche durch Reduktion des Materials. Ebenfalls ändert sich die Übergangskapazität an den Kristallübergängen, Bahnwiderstand, innerhalb des Materials in signifikanter Weise. Die Übergangskapazität an den Kontakten ändert sich dagegen kaum.

Anders reagiert der Sensor, wenn reduzierbare Gase, z. B. NO oder NO₂, auf der Oberfläche des Sensormaterials absorbiert werden. Aufgrund der geringeren Reaktivität kommt es zu einer geringeren Änderung der elektrischen Parameter der Sensormasse (Bahnwiderstand) bzw. des Sensormaterials als bei oxidierbaren Gasen. Allerdings kommt es zu einer erheblichen Beeinflussung der Schottky-Übergänge, hervorgerufen durch gasinduzierte Grenzflächenzustände.

Wird deshalb ein Sensor nur mit Gleichstrom als gasabhängiger Widerstand betrieben, so treten die Schaltkapazitäten an den Schottky-Übergängen in den Hintergrund. Es wird praktisch nur der Massewiderstand des polykristallinen Metalloxids ausgewertet.

Zu anderen und wesentlich reicheren Ergebnissen kommt man hingegen, wenn die Impendanz des Sensors im Rahmen von Auswertestrategien betrachtet wird. Dadurch stellt die Erfindung eine Vorrichtung zur Verfügung, der die simultane Detektion sowohl von oxidierbaren wie auch von reduzierbaren Substanzen mit einem einzigen Sensor ermöglicht, wobei die von der Natur des Gases abhängigen Veränderungen der vorstehend beschriebenen Kapazitäten ausgenutzt werden.

### Kurzbeschreibung der Zeichnung, in der zeigen:

- Figur 1: das elektrische Ersatzschaltbild eines Sensors mit Zinndioxid als Sensormaterial als Kombination von Widerständen und Kapazitäten
- Figur 2: ein erweitertes, genaueres elektrisches Ersatzschaltbild gemäß der Figur 1 durch die Hinzunahme von Kontaktkapazitäten und Widerstände der Kontaktelektroden
- Figur 3: zwei lmpendanzverläufe, nämlich Kurve 3.2 den lmpendanzverlauf des Kontaktüberganges und Kurve 3.1 den Impendanzverlauf innerhalb des polykristallinen Gefüges des Bahnwiderstandes mit größeren Kapazitäten
- Figur 4: das vereinfachte Ersatzschaltbild des Sensors bestehend nur aus Kondensator und Widerstand
- Figur 5: eine technische Realisierung einer Vorrichtung mit nicht zu hohem Anspruch an die Messgenauigkeit
- Figur 6: die Messergebnisse aus der Schaltung gemäß der Figur 5 in Kurvenform und
- Figur 7: eine technische Realisierung einer Vorrichtung mit hoher Messgenauigkeit, die von den Ansprüchen nicht umfasst wird.

### Wege zur Ausführung der Erfindung:

Der elektrische Widerstand eines Metalloxidsensors ergibt sich zum einen aus der Masse des sensitiven Metalloxides und dessen ohm'schen Widerstandes bzw. des spezifischen Widerstandes. Abhängig von der Korngröße und der Dicke des Materials befinden sich zwischen den einzelnen Kristallen des tatsächlich polykristallinen Metalloxides bereits Schottky-Übergänge mit Kontaktkapazitäten, die vielfach hintereinander und parallel geschaltet sind. Beim Übergang auf die Kontaktelektroden ergeben sich Übergänge, die ebenfalls als Schottky-Übergänge mit entsprechenden Schaltkapazitäten zu begreifen sind, was in Figur 1 als Ersatzschaltbild dargestellt ist.

Figur 2 zeigt ein genaueres Ersatzschaltbild des Modells gemäß Figur 1, wobei R1 der Volumenanteil des Widerstandes ist, R2 der Übergangswiderstand des Metalloxides zu den Kontakten und C2 die Kapazität an den Schottky-Übergängen; Widerstand R3 und Kondensator C3 beschreiben die gasabhängigen Diffusions- und Migrationseffekte des sensitiven Materials an den elektrischen Übergängen innerhalb des polykristallinen Materials des Sensors.

Die Kapazitäten an den Kontaktübergängen können mit 10-100pF bestimmt werden während die Kapazitäten an den Schottky-Übergängen der Korngrenzen innerhalb des Materials Werte von 0,1-2mF annehmen können, je nach Korngröße und Schichtdicke.

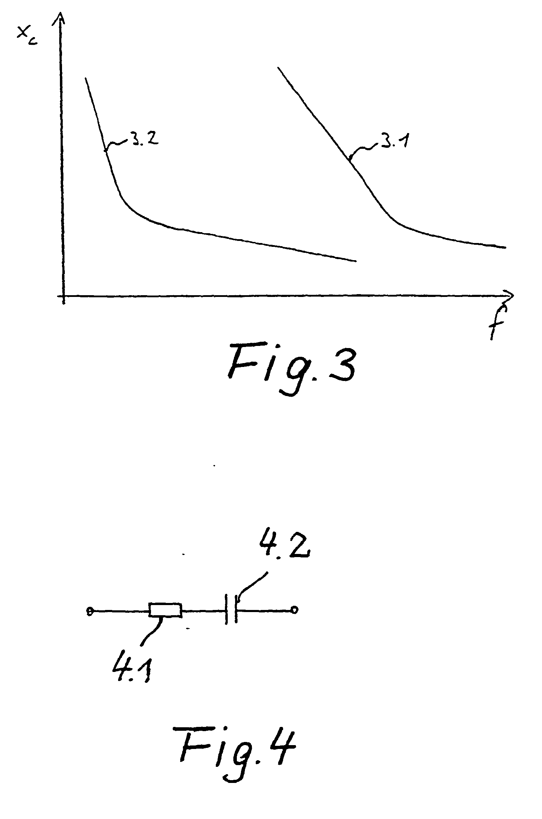

Wird entsprechend der Figur 4 das Ersatzschaltbild des Sensors grob vereinfacht bestehend aus einer Reihenschaltung eines einzigen Kondensators 4.2 und eines einzigen Widerstand 4.1 jeweils für den Kontaktübergang bzw. für die polykristalline Masse, so ergeben sich Impendanzverläufe, Kapazität über der Frequenz aufgetragen, wie sie in Figur 3 dargestellt sind. Dabei ist der Verlauf der Kurve 3.2 der Impendanzverlauf des Kontaktüberganges, dessen Kapazitäten deutlich kleiner sind und deren Kapazitäten für eine mit der Frequenz abnehmenden Impendanz bis zu einer relativ hohen Frequenz sorgen. Bei sehr hohen Frequenzen überwiegt der ohm'sche Anteil des in Reihe geschalteten Bahnwiderstandes, so dass die Kurve asymptotisch verläuft.

Die wesentlichen höheren Kapazitäten innerhalb des polykristallinen Gefüges des Bahnwiderstandes des Sensormaterials sind dafür verantwortlich, dass die Kurve 3.1 schon bei relativ niedrigen Frequenzen asymptotisch wird.

Selbstverständlich lassen sich die beiden Kurven 3.1 und 3.2 nicht ideal und getrennt darstellen wie in Figur 3 gezeigt, weil beide Effekte sich innerhalb des Sensors gleichzeitig abspielen. Vielmehr wird man stets eine Addition der Effekte feststellen.

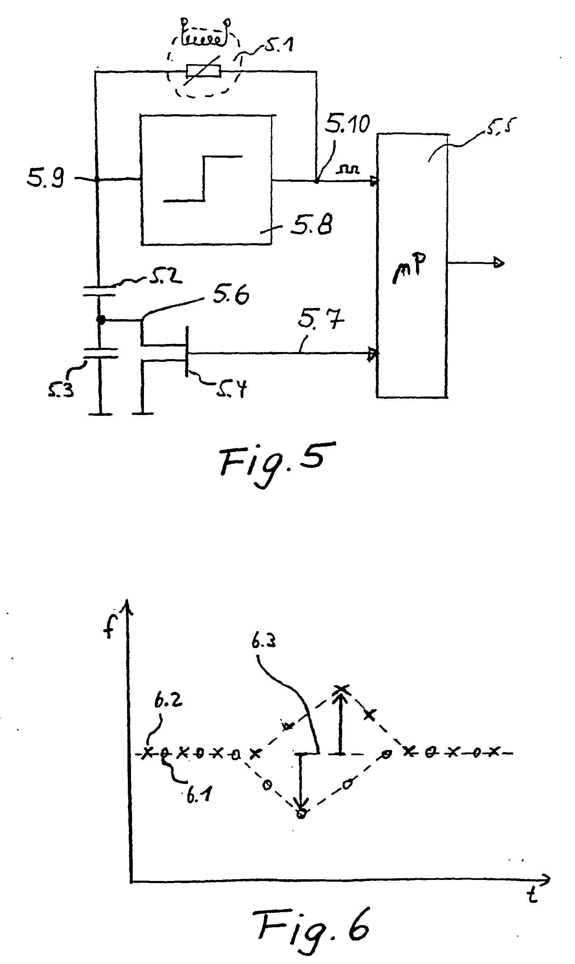

Um nun die beschriebenen Effekte zu messtechnischen Zwecken ausnutzen zu können, werden erfindungsgemäß mehrere Methoden beschrieben. Dazu zeigt Figur 5 eine technische Realisierung einer Vorrichtung mit nicht zu hohem Anspruch an die Messgenauigkeit, die für viele Zwecke bewährt und ausreichend ist. Ein auf eine Temperatur von z. B. 350 Grad C geheizter Sensor 5.1 ist Bestandteil einer Schwingschaltung 5.8, vorzugsweise ist der Sensor 5.1 mit seinen Kontaktelektroden 5.9 und 5.10 parallel zur Schwingschaltung 5.8 gelegt. Das Ausgangssignal derselben am Ausgang 5.10 wird auf einen Mikroprozessor 5.5, mP, aufgegeben. Ein Kondensator 5.2 ist mit einem Kondensator 5.3 in Reihe geschaltet und mit dem einen Eingang 5.9 der Schwingschaltung 5.8 bzw. mit der einen Kontaktelektrode 5.9 des Sensors 5.1 verbunden, wobei der Kondensator 5.3 abwechselnd bzw. periodisch kurzgeschlossen werden kann.

Das abwechselnde Kurzschließen des Kondensators 5.3 kann zum Beispiel durch einen Feldeffekttransistor 5.4 erfolgen, in dem zum Beispiel bei einem p-Kanal-Sperrschichtfet der Drain- bzw. der Sourceanschluß 5.6 mittig zwischen den Kondensatoren 5.2 und 5.3 liegt, der mit Masse verbunden ist; der Gateanschluß 5.7 ist mit dem Mikroprozessor 5.5, mP, verbunden. Der Feldeffekttransistor 5.4 wird von dem Mikroprozessor 5.5 gesteuert. Die Beschaltung ist so ausgelegt, dass bei an Normalluft angepaßtem Sensor 5.1 sich eine Frequenz von ca. 3-5 khz der Schwingschaltung 5.8 bei Kurzschluss des Kondensators 5.3 ergibt, hingegen sich bei in Reihe geschalteten Kondensatoren 5.3 und 5.2 eine Frequenz von ca. 150 kHz der Schwingschaltung 5.8 einstellt. Der interne Zähler des Mikroprozessors 5.5, mP, stellt auch die von der Schwingschaltung 5.8 abgegebene Frequenz fest. Durch kontinuierliches Schalten des Feldeffekttransistors 5.4 zwischen Kurzschluss des Kondensators 5.3 und Reihenschaltung der Kondensatoren 5.3 und 5.2 wird entsprechend die Eingangskapazität der Schwingschaltung 5.8 geändert, wodurch sich die Ausgangsfrequenz derselben verändert.

Vorteilhaft wird das Verhältnis der jeweiligen Betriebszeiten so gewählt, dass die in den Zähler des Mikroprozessors 5.5 eingelesene Zahl der Schwingungen sich in etwa entsprechen. Da die Frequenzen ein Verhältnis von ca. 1:30 haben, wird das Ansteuerverhältnis des Feldeffekttransistors bei ca. 30:1 gewählt werden.

Der Mikroprozessor liest jeweils den Zähler aus und ordnet die Ergebnisse je einem "Kanal" zu, so dass ein Bild gemäß dem in Figur 6 gezeigten entsteht. Dabei stellt die eine Kurve 6.1 die Ergebnisse der hochfrequenten Signale (o...) und die andere Kurve 6.2 die der niederfrequenten Signale (x...) - dar. Die hochfrequenten Signale 6.1 werden deutlich mehr von Stickoxiden oder anderen reduzierbaren Gasen beeinflußt als die niederfrequenten Signale 6.2, die praktisch nur von oxidierbaren Gasen beeinflußt werden. Da die Reaktionen gegenläufig sind, ist der Abstand 6.3 der Signale (- .. -) voneinander ein Maß für die Summe der anwesenden Gase, was z. B. bei Fragen der Lüftungssteuerung von Gebäuden oder von Kraftfahrzeugkabinen völlig ausreicht. So kann z. B. dann, wenn der Abstand 6.3 ein bestimmtes Größenmass überschreitet, die Lüftungsklappe eines Kraftfahrzeuges geschlossen werden. Vorteilhaft handelt es sich um ein statisches Signal, welches z. B. in Tunnelsituationen zeitlich unbegrenzt eine ungewöhnlich hohe Belastung der Luft zuverlässig detektiert.

Damit ist das erfindungsgemäße Verfahren bekannten Verfahren zur simultanen Detektion von Diesel- und Benzinabgasen insofern überlegen, weil die bekannten Verfahren dynamisch arbeiten und bei permanentem Gaspegel keine Signale mehr erzeugen und zum Beispiel in einem extrem mit Abgasen belasteten Tunnel die Lüftungsklappe wieder öffnen würde, was nicht immer erwünscht ist. Selbstverständlich ist programmtechnisch dafür Sorge getragen, dass die bei Normalluft eingelesenen Daten zur Kalibration der "Null-Linie" herangezogen werden. Auch werden die Änderungsbeträge entsprechend der Ansprechempfindlichkeit auf die jeweiligen Zielgase gewichtet, wozu entsprechende Techniken bekannt sind, weshalb im Rahmen der Erfindung darauf nicht eingegangen wird.

Bei der Beurteilung von Verbrennungsprozessen ist es dagegen von größtem Interesse, die jeweiligen Anteile oxidierbarer Gase und von Stickoxiden zu kennen. In diesem Falle kennzeichnet die Abweichung von der "Null-Linie" die Konzentration des jweiligen Gases, was vom Mikroprozessor mit einem dafür eingerichteten Programm ausgewertet werden kann.

Auch bei der On-board-Diagnose, OBD, von Fahrzeug-Katalysatoren kann das Verfahren mit Erfolg eingesetzt, da die Sensoren robust und preiswert sind und die Auswerteelektronik ebenfalls keine besonderen Anforderungen stellt.

Selbstverständlich ist das vorgestellte Schaltungsbeispiel eine von zahlreichen Möglichkeiten. Der erfindungsgemäße Grundgedanke ist es, die zur Auswertung genutzte Wechselspannung zwischen zwei Frequenzen hin und her zu schalten und die gewonnenen Daten so auszuwerten, dass sich eine Information über die Summe der anwesenden Gase angeben bzw. die Konzentration von anwesenden oxidierbaren und von reduzierbaren Gasanteilen bestimmen lässt, indem die im Vergleich zu Normalluft bei den jeweiligen Frequenzen spezifischen Änderungen mit einer geeigneten elektrischen Auswerteschaltung erfasst werden.

Bei höheren Anforderungen an die Messgenauigkeit bleibt der oben geschilderte Gedanken erhalten, die Frequenz der Betriebsspannung des Sensors zwischen den beiden genannten Frequenzen umzutasten.

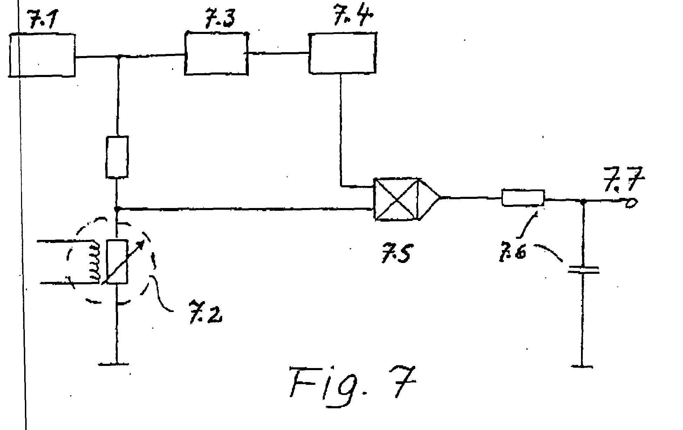

Figur 7 zeigt eine technische Realisierung einer Vorrichtung mit hoher Messgenauigkeit, die von den Ansprüchen nicht umfaßt wird. Dabei wird in weiterer Ausgestaltung des Verfahrens durch eine Phasenbetrachtung mit Hilfe einer geeigneten Vergleicherschaltung nach dem Stand der Technik jeweils für jede Frequenz das Signal in den Real- und den Scheinanteil zerlegt. Die so gewonnene Information ist bei etwas höherem Aufwand genauer als die vorstehend beschriebene Methode zu Figur 5.

Zu diesem Zweck wird Wechselspannung aus einem Generator 7.1, der Wechselspannungen verschiedener Frequenzen zu erzeugen imstande ist, einem Sensor 7.2 über einen Phasenschieber 7.3 und einem Rechteckformer 7.4 zugeführt. Das Ausgangssignal des Rechteckformers 7.4 und die über dem Sensor 7.2 abgegriffene Spannung werden einem Mischer 7.5, zum Beispiel einem Multiplizierer, zugeführt, dessen Ausgangssignal über einen Tiefpass 7.6 gemittelt wird. Die nach dem Tiefpaß 7.6 erhaltene Ausgangsspannung 7.7 stellt je nach Einstellung des Phasenschiebers 7.3 ein Maß für den Imaginärteil oder den Realteil oder für eine Mischung beider Anteile des komplexen Sensorwiderstandes dar, siehe Prinzip des phasenrichtigen Gleichrichtens pp.

Die so mögliche Trennung von Real- und lmaginärteil des komplexen Sensorwiderstandes erlaubt die Unterscheidung von kapazitiven und resistiven Effekten am Sensor 7.2. Dies ermöglicht die Unterscheidung bzw. die gleichzeitige Messung von Gasen ermöglicht, die sich durch unterschiedliche Reaktionsmechanismen an unterschiedlichen Stellen des Sensors, nämlich an den Korngrenzen oder am Metall-Halbleiter-Kontakt, unterscheiden. Zudem können bei der Adsorption von Gasen auf HL-Sensoren mehrere Adsorptions- und Umladungseffekte mit zum Teil gegenläufigen Auswirkungen auf den Realteil des Sensorwiderstandes auftreten, was die schnelle und sichere Detektion des Gases erschwert.

Es ist möglich, ein Gemisch verschiedener Frequenzen in Bezug auf Änderungen der Phasenlage zueinander im Sinne vorstehender Lehre zu nutzen. Zuletzt ist es denkbar, die Frequenzen gleitend durchzustimmen und gleitend das Signal in einen Imaginär- und/oder Realteil zu analysieren und aufzutragen, bzw. der weiteren Verarbeitung zuzuführen.

Es liegt auf der Hand, dass die Aussagefähigkeit einer Sensoranordnung erheblich verbessert werden kann, wenn das beschriebene Verfahren mit mehreren Sensorelementen gleichzeitig eingesetzt wird. Wenn die Sensorelemente mit unterschiedlicher Temperatur betrieben werden oder aus unterschiedlichen sensitiven Materialien bestehen, wird eine Fülle von Informationen von den Sensoren abgegeben, die nach dem Prinzip der Mustererkennung oder mit Hilfe artifizieller neuronaler Netze bestimmten Zielgasen zugeordnet werden können.

Speziell für die Anwendung zur Abgasbewertung hinter Feuerungsanlagen und bei der Beurteilung der Wirksamkeit von Katalysatoren in Kraftfahrzeugen ist in der Regel keine besonders hohe Reaktionsgeschwindigkeit gefragt. Daher erhöht es die Selektivität gegenüber den einzelnen Gasgruppen, oxidierbar oder reduzierbar, wenn neben der Impendanzanalyse mit Hilfe unterschiedlicher Frequenzen auch die Temperatur des Sensorelementes verändert wird. Bei höheren Temperaturen, zum Beispiel ca. >350 Grad C bei Zinndioxid-Sensoren, steigt die Empfindlichkeit gegenüber oxidierbaren Gasen wie Kohlenmonoxid oder Kohlenwasserstoffen und deren Fragmenten an und die Empfindlichkeit gegenüber z. B. Stickoxiden sinkt. Umgekehrt wird bei niedrigen Temperaturen von zum Beispiel 150 Grad C die Empfindlichkeit gegenüber zum Beispiel Stickoxiden sehr hoch, während die Empfindlichkeit gegenüber Kohlenmonoxid etc. sinkt. Dieser Effekt wird durch die vorstehend beschriebenen Methoden erheblich unterstützt. Neben der Selektivität wächst die Stabilität der Anordnung, da bei den niedrigen Temperaturen zugeordneten hohen Frequenzen keine Migrationseffekte und kein lonentransport mehr zu beobachten ist und auch eventuell eingelagerte Wassermoleküle nicht mehr elektrisch dissoziiert werden, was der Lebensdauer und der Stabilität der Senorelemente zugute kommt.

Beim Einsatz zur Beurteilung der Verbrennungsprozesse in Feuerungsanlagen oder beim Einsatz zur On-board-Diagnose zur Beurteilung der Effektivität von Kraftfahrzeug-Katalysatoren sind Sensormaterialien vorteilhaft einsetzbar, bei denen eine höhere Arbeitstemperatur möglich ist. Vorteilhaft erprobt sind Mischoxide mit hohem Anteil von Wolframtrioxid, Galliumoxid. Vanadate und Molybdate sind als Beimengungen ebenfalls erprobt worden. Bei hohen Arbeitstemperaturen und genannten sensitiven Substanzen wird insbesondere die geringe Querempfindlichkeit gegenüber Wasser vorteilhaft erwähnt.

Allen genannte Verfahren ist gemeinsam, dass die sensorinternen Kapazitäten und deren Einfluss auf die Impendanz des Sensors als komplexes Bauteil bei Wechselströmen unterschiedlicher Frequenz zur Gewinnung von Informationen ausgenutzt werden.

### Gewerbliche Anwendbarkeit:

Das Verfahren und die Vorrichtung sind bei der quantitativen und qualitativen Feststellung von Gasen einsetzbar, die oxidierbar oder reduzierbar sind, insbesondere zum Zwecke der Steuerung von Lüftungseinrichtungen in Gebäuden oder Fahrzeugen und zum Zwecke der Überwachung von Verbrennungsprozessen und von Abgaskatalysatoranlagen. Die Nützlichkeit der Erfindung besteht insbesondere darin, dass aufgrund der Veränderung des elektrischen Bahnwiderstandes des Sensors eine Aussage zur Anwesenheit und zur Konzentration oxidierbarer Gase und aufgrund der Veränderung des elektrischen Kontaktwiderstandes eine Aussage zur Anwesenheit und zur Konzentration reduzierbarer Gase mit Hilfe der elektrischen Auswerteeinrichtung gemacht werden kann.

## Patentansprüche

1. Verfahren zur Detektion oxidierbarer und reduzierbarer Gase in der Luft, insbesondere NO₂ sowie CO, zum Zwecke der Steuerung von Lüftungseinrichtungen in Gebäuden oder Fahrzeugen und zum Zwecke der Überwachung von Verbrennungsprozessen oder von Abgaskatalysatoranlagen, unter Verwendung mindestens eines mit Kontaktelektroden (5.9;5.10) versehenen, stromdurchflossenen und beheizbaren Sensors (5.1) mit einem Sensormaterial aus Metalloxid sowie unter Verwendung einer elektrischen Auswerteschaltung, wobei der Sensor (5.1) von Wechselstrom durchflossen wird, der zwischen mindestens zwei Frequenzen umgeschaltet wird, und einerseits die Änderung der Kapazitäten zwischen dem Sensormaterial und den Kontaktelektroden (5.9;5.10) von der Auswerteschaltung als Merkmal der Anwesenheit von reduzierbaren Gasen und andererseits die Änderung der Kapazitäten Innerhalb der Masse des Sensormaterials als Merkmal der Anwesenheit von oxidierbaren Gasen ausgewertet wird, wobei die unterschiedlichen Frequenzen (fx:fy) mittels frequenzbestimmenden Bauteilen und mit einer Schwingschaltung (5.8) erzeugt werden, und der Sensor (5.1) Bestandteil der Schwingschaltung (5.8) ist, und die Schwingung der Schwingschaltung (5.8) durch abwechselndes Umschalten der frequenzbestimmenden Bauteile (5.2;5.3) zwischen mindestens den beiden Frequenzen (fx;fy) abwechselnd verändert wird und die diesen Frequenzen (fx;fy) zugeordneten Ausgangssignale der Schwingschaltung (5.8), wenn der Sensor (5.1) von Normalluft umspült wird, zur Kalibration je einer Nulllinie herangezogen werden, wobei der höheren Frequenz (fy) der beiden Frequenzen (fx;fy) stets reduzierbare Gase und der niedrigeren Frequenz (fx) der beiden Frequenzen (fx,fy) stets oxidierbare Gase zugeordnet werden, und, wenn der Sensor (5.1) von einem Gas abweichend von Normalluft umspült wird, sich die Ausgangssignale der Schwingschaltung (5.8) gegenüber der jeweiligen Nulllinie verändern, wobei bei einer Abweichung der höheren Frequenz (fy) das Vorhandensein von reduzierbaren Gasen und bei einer Abweichung der niederen Frequenz (fx) das Vorhandensein von oxidierbaren Gasen im Bereich des Sensors (5.1) nachgewiesen wird, und die jeweiligen Frequenzabweichungen als Maß für die Konzentration anwesender Gase herangezogen werden, wobei
der beheizte Sensor (5.1) mit seinen Kontaktelektroden (5.9;5.10) parallel der Schwingschaltung (5.8) gelegt ist, deren Ausgangssignal auf den Mikroprozessor (mP, 5.5) gegeben wird, wobei als frequenzbestimmende Bauteile zwei in Reihe geschaltete Kondensatoren (5.2;5.3) dienen, von denen der zweite Kondensator (5.2) auf den Eingang (5.9) der Schwingschaltung (5.8) gelegt ist und auf diesen arbeitet, und der erste Kondensator (5.3) zur Veränderung der Eingangskapazität der Schwingschaltung (5.8) abwechselnd oder periodisch kurzgeschlossen wird.

2. Verfahren nach Anspruch 1, **dadurch gekennzeichnet,**
**dass** das abwechselnde Kurzschließen des ersten Kondensators (5,3) durch einen Feldeffekttransistor (5.4) in Zusammenhang mit dem Mikroprozessor (5.5,mP) erfolgt, in dem der Drain- oder der Sourceanschluß (5.6) des Feldeffekttransistors (5.4) mittig zwischen den beiden Kondensatoren (5.2) und (5.3) liegt, wobei der erste Kondensator (5.3) mit Masse und der Gateanschluß (5.7) des Feldeffekttransistors (5.4) mit dem Mikroprozessor (5.5,mP) verbunden ist, und der Feldeffekttransistor 5.4 von dem Mikroprozessor (5.5,mP) gesteuert wird.

3. Verfahren nach Anspruch 2, **dadurch gekennzeichnet,**
**dass** die Beschaltung so ausgelegt ist, dass bei an Normaltuft angepaßtem Sensor (5.1) sich eine Frequenz von ca. 3-5 kHz der Schwingschaltung (5.8) bei Kurzschluss des ersten Kondensators (5.3) einstellt, hingegen sich bei in Reihe geschalteten beiden Kondensatoren (5.3,5.2) eine Frequenz von ca. 150 kHz der Schwingschaltung (5.8) einstellt, wobei durch ein kontinuierliches Schalten des Feldeffekttransistors (5.4) zwischen Kurzschluss des ersten Kondensators (5.3) und Reihenschaltung der beiden Kondensatoren (5.3,5.2) entsprechend die Eingangskapazität der Schwingschaltung (5.8) geändert wird, wodurch sich die Ausgangsfrequenz der Schwingschaltung (5.8) verändert.

4. Vorrichtung zur Detektion oxidierbarer und reduzierbarer Gase In der Luft zur Durchführung des Verfahrens gemäß Anspruch 1, mindestens einem mit Kontaktelektroden (5.9;5.10) versehenen, stromdurchflossenen und beheizbaren Sensor (5.1;7.2) mit einem Sensormaterial aus Metalloxid sowie mit einer elektrischen Auswerteschaltung, wobei der Sensor (5.1) von Wechselstrom durchflossen wird, der zwischen mindestens zwei Frequenzen umgeschaltet wird, und einerseits die Änderung der Kapazitäten zwischen dem Sensormaterial und den Kontaktelektroden (5.9;5.10) von der Auswerteschaltung als Merkmal der Anwesenheit von reduzierbaren Gasen und andererseits die Änderung der Kapazitäten innerhalb der Masse des Sensormaterials als Merkmal der Anwesenheit von oxidierbaren Gasen ausgewertet wird, wobei die unterschiedlichen Frequenzen (fx;fy) mittels frequenzbestimmenden Bauteilen und mit einer Schwingschaltung (5.8) erzeugt werden, der Sensor (5.1;7.1) Bestandteil der Schwingschaltung (5.8) ist, und die Schwingung der Schwingschaltung (5.8) durch abwechselndes Umschalten der frequenzbestimmenden Bauteile (5.2;5.3) zwischen mindestens den beiden Frequenzen (fx;fy) abwechselnd verändert wird und wobei programmtechnisch dafür Songe getragen ist, dass die Frequenzen (fx;fy) zugeordneten Ausgangssignale der Schwingschaltung (5.8), wenn der Sensor (5.1) von Normalluft umspült wird, zur Kalibration je einer Nulllinie herangezogen werden, wobei der höheren Frequenz (fy) der beiden Frequenzen (fx;fy) stets reduzierbare Gase und der niedrigeren Frequenz (fx) der beiden Frequenzen (fx,fy) stets oxidierbare Gase zugeordnet werden, und, wenn der Sensor (5.1) von einem Gas abweichend von Normalluft umspült wird, sich die Ausgangssignale der Schwingschaltung (5.8) gegenüber der jeweiligen Nulllinie verändern, wobei bei einer Abweichung der höheren Frequenz (fy) das Vorhandensein von reduzierbaren Gasen und bei einer Abweichung der niederen Frequenz (fx) das Vorhandensein von oxidierbaren Gasen im Bereich des Sensors (5.1) nachgewiesen wird, und die jeweiligen Frequenzabweichungen als Maß für die Konzentration anwesender Gase herangezogen werden,
wobei ein beheizbarer Sensor (5.1) Bestandteil einer Schwingschaltung (5.8) ist, wobei der Sensor (5.1) mit seinen Kontaktelektroden (5.9,5.10) parallel zur Schwingschaltung (5.8) gelegt ist, an deren Ausgang (5.10) ein Ausgangssignal erscheint, welches auf einen Mikroprozessor (5.5,mP) aufgegeben wird, wobei ein erster Kondensator (5.3) mit einem zweiten Kondensator (5.2) in Reihe geschaltet ist welcher mit dem einen Eingang (5.9) der Schwingschaltung (5.8) sowie mit der einen Kontaktelektrode (5.9) des Sensors (5.1) verbunden ist, wobei der erste Kondensator (5.3) abwechselnd oder periodisch kurzgeschlossen werden kann.

5. Vorrichtung nach Anspruch 4, **dadurch gekennzeichnet,**
**dass** das abwechselnde Kurzschließen des ersten Kondensators (5,3) durch einen Feldeffekttransistor (5.4) in Zusammenhang mit dem Mikroprozessor (5.5,mP) erfolgt, in dem der Drain- oder der Sourceanschluß (5.6) des Feldeffekttransistors (5.4) mittig zwischen den beiden Kondensatoren (5.2) und (5.3) liegt, wobei der erste Kondensator (5.3) mit Masse und der Gateanschluß (5.7) des Feldeffekttransistors (5.4) mit dem Mikroprozessor (5.5,mP) verbunden ist, wobei der Feldeffekttransistor 5.4 von dem Mikroprozessor (5.5,mP) gesteuert wird,

6. Vorrichtung nach Anspruch 5, **dadurch gekennzeichnet,**
**dass** die Beschaltung so ausgelegt ist, dass bei an Normalluft angepaßtem Sensor (5.1) sich eine Frequenz von ca. 3-5 kHz der Schwingschaltung (5,8) bei Kurzschluss des ersten Kondensators (5.3) ergibt, hingegen sich bei in Reihe geschalteten beiden Kondensatoren (5.3,5.2) eine Frequenz von ca. 150 kHz der Schwingschaltung (5.8) einstellt, wobei durch ein kontinuierliches Schalten des Feldeffekttransistors (5.4) zwischen Kurzschluss des ersten Kondensators (5.3) und Reihenschaltung der beiden Kondensatoren (5.3,5.2) entsprechend die Eingangskapazität der Schwingschaltung (5.8) geändert wird, wodurch sich die Ausgangsfrequenz der Schwingschaltung (5.8) verändert.

7. Vorrichtung nach einem der vorherigen Ansprüche 4 bis 6,
**dadurch gekennzeichnet, dass** der interne Zähler des Mikroprozessors (5.5.mP) die von der Schwingschaltung (5.8) abgegebene Frequenz feststellt.

## Claims

1. A method for detecting oxidizable and reducible gases in the air, especially NO₂ and CO, for purposes of controlling ventilation systems in buildings or vehicles and for purposes of monitoring combustion processes or catalytic converter systems, employing at least one heatable sensor (5.1) through which current flows and that is provided with contact electrodes (5.9; 5.10), said sensor being made of metal oxide, and also employing an electric evaluation circuit, whereby alternating current that is switched over between at least two frequencies flows through the sensor (5.1) and, on the one hand, the change in the capacitances between the sensor material and the contact electrodes (5.9; 5.10) is evaluated by the evaluation circuit as an indication of the presence of reducible gases and, on the other hand, the change in the capacitances within the mass of the sensor material is evaluated as an indication of the presence of oxidizable gases, whereby the differing frequencies (fx; fy) are generated by means of frequency-determining components and with an oscillation circuit (5.8), and the sensor (5.1) is an integral part of the oscillation circuit (5.8), and the oscillation of the oscillation circuit (5.8) is alternatingly changed between at least the two frequencies (fx; fy) by alternatingly switching over between the frequency-determining components (5.2; 5.3), and the output signals of the oscillation circuit (5.8) that are associated with these frequencies (fx; fy) are each employed for the calibration of a zero line when the sensor (5.1) is flooded with normal air, whereby reducible gases are always associated with the higher frequency (fy) of the two frequencies (fx; fy) and oxidizable gases are always associated with the lower frequency (fx) of the two frequencies (fx; fy) and, when the sensor (5.1) is flooded with a gas that differs from normal air, the output signals of the oscillation circuit (5.8) change with respect to the appertaining zero line, whereby the presence of reducible gases is confirmed in case of a divergence from the higher frequency (fy) while the presence of oxidizable gases is confirmed in case of a divergence from the lower frequency (fx) in the area of the sensor (5.1), and the appertaining frequency divergences are employed as a measure of the concentration of the gases present, whereby the heated sensor (5.1) with its contact electrodes (5.9; 5.10) is connected in parallel to the oscillation circuit (5.8) whose output signal is conveyed to the microprocessor (mP, 5.5), whereby two capacitors (5.2; 5.3) connected in series serve as the frequency-determining components, the second capacitor (5.2) being connected to the input (5.9) of the oscillation circuit (5.8) and acting upon the latter, and the first capacitor (5.3) being alternatingly or periodically short-circuited in order to change the input capacitance of the oscillation circuit (5.8).

2. The method according to Claim 1, **characterized in that**
the alternating short-circuiting of the first capacitor (5.3) is carried out by a field-effect transistor (5.4) in conjunction with the microprocessor (5.5, mP) in which the drain terminal or source terminal (5.6) of the field-effect transistor (5.4) lies in the center between the two capacitors (5.2) and (5.3), whereby the first capacitor (5.3) is connected to the ground and the gate terminal (5.7) of the field-effect transistor (5.4) is connected to the microprocessor (5.5, mP), and the field-effect transistor (5.4) is controlled by the microprocessor (5.5, mP).

3. The method according to Claim 2, **characterized in that**
the circuitry is configured in such a way that a frequency of about 3 to 5 kHz is established in the oscillation circuit (5.8) when the first capacitor (5.3) is short-circuited in the case of a sensor (5.1) adapted to normal air, in contrast to which a frequency of about 150 kHz is established in the oscillation circuit (5.8) when both capacitors (5.3, 5.2) are connected in series, whereby the input capacitance of the oscillation circuit (5.8) is changed by a continuous switching of the field-effect transistor (5.4) between the short-circuit of the first capacitor (5.3) and the series connection of the two capacitors (5.3, 5.2), as a result of which the output frequency of the oscillation circuit (5.8) changes.

4. A device for detecting oxidizable and reducible gases in the air for executing the method according to Claim 1, having at least one heatable sensor (5.1; 7.2) through which current flows and that is provided with contact electrodes (5.9; 5.10), said sensor being made of metal oxide, and also employing an electric evaluation circuit, whereby alternating current that is switched over between at least two frequencies flows through the sensor (5.1) and, on the one hand, the change in the capacitances between the sensor material and the contact electrodes (5.9; 5.10) is evaluated by the evaluation circuit as an indication of the presence of reducible gases and, on the other hand, the change in the capacitances within the mass of the sensor material is evaluated as an indication of the presence of oxidizable gases, whereby the differing frequencies (fx; fy) are generated by means of frequency-determining components and with an oscillation circuit (5.8), and the sensor (5.1; 7.1) is an integral part of the oscillation circuit (5.8), and the oscillation of the oscillation circuit (5.8) is alternatingly changed between at least the two frequencies (fx; fy) by alternatingly switching over between the frequency-determining components (5.2; 5.3), and whereby a program is employed to ensure that the output signals of the oscillation circuit (5.8) that are associated with these frequencies (fx; fy) are each employed for the calibration of a zero line when the sensor (5.1) is flooded with normal air, whereby oxidizable gases are always associated with the higher frequency (fy) of the two frequencies (fx; fy) and oxidizable gases are always associated with the lower frequency (fx) of the two frequencies (fx; fy) and, when the sensor (5.1) is flooded with a gas that differs from normal air, the output signals of the oscillation circuit (5.8) change with respect to the appertaining zero line, whereby the presence of reducible gases is confirmed in case of a divergence from the higher frequency (fy) while the presence of oxidizable gases is confirmed in case of a divergence from the lower frequency (fx) in the area of the sensor (5.1), and the appertaining frequency divergences are employed as a measure of the concentration of the gases present, whereby a heatable sensor (5.1) is an integral part of an oscillation circuit (5.8), whereby the sensor (5.1) with its contact electrodes (5.9; 5.10) is connected in parallel to the oscillation circuit (5.8) on whose output (5.10) an output signal appears that is conveyed to a microprocessor (mP, 5.5), whereby a first capacitor (5.3) is connected in series to a second capacitor (5.2) that is connected to the one input (5.9) of the oscillation circuit (5.8) as well as to one contact electrode (5.9) of the sensor (5.1), whereby the first capacitor (5.3) can be alternatingly or periodically short-circuited.

5. The device according to Claim 4, **characterized in that**
the alternating short-circuiting of the first capacitor (5.3) is carried out by a field-effect transistor (5.4) in conjunction with the microprocessor (5.5, mP) **in that** the drain terminal or source terminal (5.6) of the field-effect transistor (5.4) lies in the center between the two capacitors (5.2) and (5.3), whereby the first capacitor (5.3) is connected to the ground and the gate terminal (5.7) of the field-effect transistor (5.4) is connected to the microprocessor (5.5, mP), and the field-effect transistor (5.4) is controlled by the microprocessor (5.5, mP).

6. The device according to Claim 5, **characterized in that**
the circuitry is configured in such a way that a frequency of about 3 to 5 kHz is established in the oscillation circuit (5.8) when the first capacitor (5.3) is short-circuited in the case of a sensor (5.1) adapted to normal air, in contrast to which a frequency of about 150 kHz is established in the oscillation circuit (5.8) when both capacitors (5.3, 5.2) are connected in series, whereby the input capacitance of the oscillation circuit (5.8) is changed by a continuous switching of the field-effect transistor (5.4) between the short-circuit of the first capacitor (5.3) and the series connection of the two capacitors (5.3, 5.2), as a result of which the output frequency of the oscillation circuit (5.8) changes.

7. The device according to any of the preceding Claims 4 to 6,
**characterized in that**
the internal counter of the microprocessor (5.5, mP) determines the frequency generated by the oscillation circuit (5.8).

## Revendications

1. Procédé de détection de gaz oxydables et réductibles dans l'air, en particulier NO₂ ainsi que CO, dans le but de contrôler des systèmes de ventilation dans des bâtiments ou des véhicules et dans le but de surveiller des processus de combustion ou des pots catalytiques, ayant recours à au moins un capteur (5.1) parcouru par un courant et pouvant être chauffé, doté d'électrodes de contact (5.9 ; 5.10), en un matériau de capteur en oxyde métallique, ainsi qu'ayant recours à un circuit d'évaluation électrique, le capteur (5.1) étant parcouru par un courant alternatif qui est commuté entre au moins deux fréquences et le circuit d'évaluation évaluant d'une part le changement des capacités entre le matériau capteur et les électrodes de contact (5.9 ; 5.10) comme caractéristique de la présence de gaz réductibles et d'autre part le changement des capacités à l'intérieur de la masse du matériau de capteur comme caractéristique de la présence de gaz oxydables, les fréquences différentes (fx ; fy) étant produites au moyen de composants déterminant la fréquence et avec un circuit oscillant (5.8), et le capteur (5.1) étant un élément constitutif du circuit oscillant (5.8) et l'oscillation du circuit oscillant (5.8) variant alternativement par la commutation alternante des composants (5.2 ; 5.3) déterminant la fréquence entre au moins les deux fréquences (fx ; fy), et les signaux de sortie du circuit oscillant (5.8) assignés à ces fréquences (fx ; fy) servant, lorsque le capteur baigne dans de l'air normal, à la calibration d'une ligne de zéro respectivement, des gaz réductibles étant toujours assignés à la fréquence la plus élevée (fy) des deux fréquences (fx ; fy) et des gaz oxydables à la fréquence la plus basse (fx) des deux fréquences (fx ; fy), et, lorsque le capteur (5.1) baigne dans un gaz divergeant de l'air normal, les signaux de sortie du circuit oscillant (5.8) varient par rapport à la ligne de zéro respective, un écart de la fréquence plus élevée (fy) démontrant la présence de gaz réductibles et un écart de la fréquence plus basse (fx) la présence de gaz oxydables dans la zone du capteur (5.1), et les écarts de fréquence respectifs servant de mesure pour la concentration des gaz présents,
le capteur chauffé (5.1) étant placé avec ses électrodes de contact (5.9 ; 5.10) parallèlement au circuit oscillant (5.8) dont le signal de sortie est donné au microprocesseur (mP, 5.5), deux condensateurs couplés en série (5.2 ; 5.3) servant de composants déterminant la fréquence, le deuxième condensateur (5.2) de ces deux condensateurs étant placé sur l'entrée (5.9) du circuit oscillant (5.8) et travaillant sur celui-ci, et le premier condensateur (5.3) étant mis en court-circuit alternativement ou périodiquement pour le changement de la capacité d'entrée du circuit oscillant (5.8).

2. Procédé selon la revendication 1, **caractérisé en ce que**,
le court-circuitage alternatif du premier condensateur (5.3) est produit en liaison avec le microprocesseur (5.5, mP) par un transistor à effet de champ (5.4), la borne de source ou la borne de drain (5.6) du transistor à effet de champ (5.4) étant placée de façon centrale entre les deux condensateurs (5.2) et (5.3), le premier condensateur (5.3) étant relié à la masse et la borne de grille (5.7) du transistor à effet de champ (5.4) au microprocesseur (5.5, mP), et le transistor à effet de champ (5.4) étant piloté par le microprocesseur (5.5, mP).

3. Procédé selon la revendication 2, **caractérisé en ce que**,
le câblage est conçu de sorte que pour un capteur (5.1) adapté à de l'air normal, une fréquence du circuit oscillant (5.8) d'environ 3 à 5 kHz s'établit lors du court-circuitage du premier transistor (5.3), par contre, dans le cas de deux condensateurs (5.3, 5.2) couplés en série, une fréquence du circuit oscillant (5.8) d'environ 150 kHz s'établit, un couplage continu du transistor à effet de champ (5.4) entre la mise en court-circuit du premier condensateur (5.3) et le couplage en série des deux condensateurs (5.3, 5.2) changeant de façon équivalente la capacité d'entrée du circuit oscillant (5.8), ce par quoi la fréquence de sortie du circuit oscillant (5.8) change.

4. Dispositif de détection de gaz oxydables et réductibles dans l'air pour l'application du procédé selon la revendication 1, comportant au moins un capteur (5.1 ; 7.2) parcouru par un courant et pouvant être chauffé, doté d'électrodes de contact (5.9 ; 5.10), en un matériau de capteur en oxyde métallique, comportant aussi un circuit d'évaluation électrique, le capteur (5.1) étant parcouru par un courant alternatif qui est commuté entre au moins deux fréquences et le circuit d'évaluation évaluant d'une part le changement des capacités entre le matériau de capteur et les électrodes de contact (5.9 ; 5.10) comme caractéristique de la présence de gaz réductibles, et d'autre part le changement des capacités à l'intérieur de la masse du matériau de capteur comme caractéristique de la présence de gaz oxydables, les différentes fréquences (fx ; fy) étant produites au moyen de composants déterminant la fréquence et avec un circuit oscillant (5.8), le capteur (5.1 ; 7.1) étant un élément constitutif du circuit oscillant (5.8), et l'oscillation du circuit oscillant (5.8) changeant alternativement entre au moins les deux fréquences (fx ; fy) par la commutation alternative des composants déterminant les fréquences (5.2 ; 5.3), le programme faisant techniquement en sorte que les signaux de sortie du circuit oscillant (5.8) assignés à ces fréquences (fx ; fy), lorsque le capteur (5.1) baigne dans de l'air normal, servent à la calibration d'une ligne de zéro respectivement, des gaz réductibles étant toujours assignés à la fréquence la plus élevée (fy) des deux fréquences (fx ; fy) et des gaz oxydables à la fréquence la plus basse (fx) des deux fréquences (fx ; fy) et, lorsque le capteur (5.1) baigne dans un gaz divergeant de l'air normal, les signaux de sortie du circuit oscillant (5.8) varient par rapport à la ligne de zéro respective, un écart de la fréquence plus élevée (fy) démontrant la présence de gaz réductibles et un écart de la fréquence plus basse (fx) la présence de gaz oxydables dans la zone du capteur (5.1) et les écarts de fréquence respectifs servant de mesure pour la concentration des gaz présents,
un capteur pouvant être chauffé (5.1) étant un élément constitutif d'un circuit oscillant (5,8), le capteur (5.1) étant placé avec ses électrodes de contact (5.9 ; 5.10) parallèlement au circuit oscillant (5.8) à la sortie (5.10) duquel se produit un signal de sortie, lequel est donné sur un microprocesseur (5.5, mP), un premier condensateur (5.3) étant couplé en série avec un deuxième condensateur (5.2), lequel est relié à l'entrée (5.9) du circuit oscillant (5.8), ainsi qu'avec l'électrode de contact (5.9) du capteur (5.1), le premier condensateur (5.3) pouvant être mis en court-circuit alternativement ou périodiquement.

5. Dispositif selon la revendication 4, **caractérisé en ce que**
la mise en court-circuit en alternance du premier condensateur (5.3) est produite en liaison avec le microprocesseur (5.5, mP) par un transistor à effet de champ (5.4), la borne de source ou la borne de drain (5.6) du transistor à effet de champ (5.4) étant placée de façon centrale entre les deux condensateurs (5.2) et (5.3), le premier condensateur (5.3) étant relié à la masse et la borne de grille (5.7) du transistor à effet de champ (5.4) avec le microprocesseur (5.5, mP), le transistor à effet de champ (5.4) étant piloté par le microprocesseur (5.5, mP).

6. Dispositif selon la revendication 5, **caractérisé en ce que**
le câblage est conçu de sorte que pour un capteur (5.1) adapté à de l'air normal, une fréquence du circuit oscillant (5.8) d'environ 3 à 5 kHz s'établit lors du court-circuitage du premier condensateur (5.3), alors que dans le cas des deux condensateurs (5.3, 5.2) couplés en série, une fréquence du circuit oscillant (5.8) d'environ 150 kHz s'établit, un couplage continu du transistor à effet de champ (5.4) entre la mise en court-circuit du premier condensateur (5.3) et le couplage en série des deux condenstauer (5.3, 5.2) changeant de façon équivalente la capacité d'entrée du circuit oscillant (5.8), ce par quoi la fréquence de sortie du circuit oscillant (5.8) change.

7. Dispositif selon l'une des revendications 4 à 6 ci-avant,
**caractérisé en ce que** le compteur interne du microprocesseur (5.5, mP) détermine la fréquence émise par le circuit oscillant (5.8).
